(19) Europäisches Patentamt
European Patent Office
Office européen des brevets

(11) EP 0 522 915 B1

(12) **FASCICULE DE BREVET EUROPEEN**

(45) Date de publication et mention de la délivrance du brevet:
**13.03.1996 Bulletin 1996/11**

(51) Int Cl.[6]: **C07D 239/42**, A61K 31/505

(21) Numéro de dépôt: **92401772.6**

(22) Date de dépôt: **24.06.1992**

(54) **Dérivés de pyrimidine-4-carboxamide, leur préparation et leur application en thérapeutique**

Pyrimidin-4-carboxamidderivate, ihre Herstellung und ihre Verwendung in der Therapie

Pyrimidine-4-carboxamide derivatives, their preparation and their use in therapeutics

(84) Etats contractants désignés:
**AT BE CH DE DK ES FR GB GR IT LI LU MC NL PT SE**

(30) Priorité: **27.06.1991 FR 9107937**

(43) Date de publication de la demande:
**13.01.1993 Bulletin 1993/02**

(73) Titulaire: **SYNTHELABO**
**F-92350 Le Plessis Robinson (FR)**

(72) Inventeurs:
- **George, Pascal**
  **F-78730 St Arnoult en Yvelines (FR)**
- **Froissant, Jacques**
  **F-41160 Morée (FR)**
- **Tixidre, Arlette**
  **F-91400 Orsay (FR)**

(74) Mandataire: **Ludwig, Jacques et al**
**SYNTHELABO,**
**Service Brevets,**
**B.P. 72**
**F-92352 Le Plessis Robinson Cédex (FR)**

(56) Documents cités:

| | |
|---|---|
| **EP-A- 0 301 936** | **EP-A- 0 307 303** |
| **EP-A- 0 435 749** | **DE-A- 2 143 730** |

EP 0 522 915 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

## Description

La présente invention a pour objet des dérivés de pyrimidine-4-carboxamide, leur préparation et leur application en thérapeutique.

Les composés de l'invention répondent à la formule générale (I)

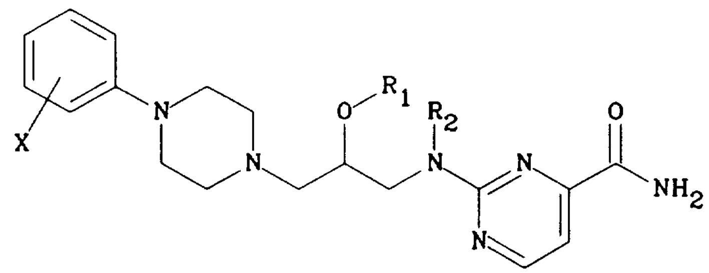

(I)

dans laquelle

X représente un ou plusieurs atomes et/ou groupes choisis parmi les suivants : hydrogène, fluor, chlore, méthoxy et cyclopropyle,
$R_1$ représente un atome d'hydrogène
et
$R_2$ représente un atome d'hydrogène ou un groupe méthyle.

Les demandes de brevets EP-0 307 303, EP-0 301 936 et DE-2 143 730 décrivent des composés ayant des structures chimiques plus ou moins proches de celle des composés de l'invention, mais n'ayant pas leurs propriétés utiles dans le traitement d'affections du bas appareil urinaire. La demande de brevet EP-0 435 749, publiée après la date de priorité de la présente demande, décrit des composés ayant une structure chimique proche de celle des composés de l'invention, et ayant les mêmes applications thérapeutiques.

Les composés de l'invention peuvent exister, sous la forme de racémates ou d'énantiomères, à l'état de bases libres ou de sels d'addition à des acides pharmaceutiquement acceptables.

Conformément à l'invention, on peut préparer les composés de formule générale (I) selon le procédé du schéma 1 qui suit. on fait réagir une pipérazine de formule générale (II) dans laquelle X est tel que défini ci-dessus, avec un réactif époxyde de formule (III), dans laquelle Y représente un

Schéma 1

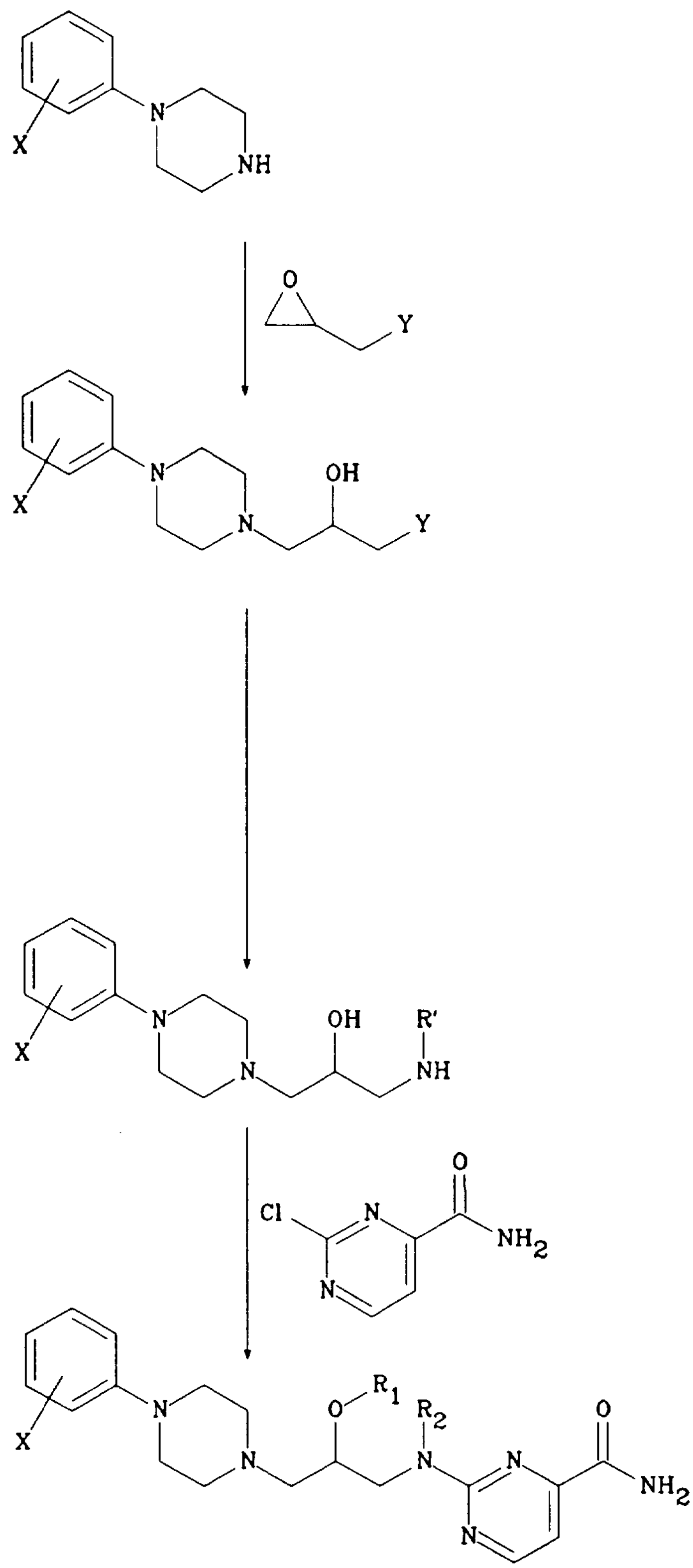

(II)

(III)

(IVa)

(V)

(VI)

(I)

groupe phtalimido. La réaction est effectuée dans un solvant protique tel qu'un alcool aliphatique, par exemple le propan-2-ol, à une température de 20 à 100°C.

On obtient un dérivé de l-aminopropan-2-ol de formule générale (IVa) que l'on traite avec de l'hydrate d'hydrazine dans un solvant protique tel qu'un alcool aliphatique, par exemple l'éthanol, à une température de 20 à 80°C, puis par de l'acide chlorhydrique aqueux à une température de 80 à 100°C pour obtenir le chlorhydrate de l'amine de formule

générale (V), selon les conditions classiques de transformation du phtalimide substitué en amine. On fait ensuite réagir le composé de formule générale (V), dans laquelle R' représente l'hydrogène, avec le 2-chloropyrimidine-4-carboxamide de formule (VI), dans un solvant aprotique, par exemple le *N,N*-diméthylformamide, en présence d'une base, par exemple le carbonate de potassium, à une température de 20 à 60°C pour obtenir un composé de formule générale (I) dans laquelle $R_1$ et $R_2$ sont chacun un atome d'hydrogène.

On obtient les composés de formule générale (I) dans laquelle $R_2$ représente un groupe méthyle en traitant un aminoalcool de formule générale (V), dans laquelle R' représente l'hydrogène, par un formiate d'alkyle, par exemple le formiate d'éthyle, à une température de 40 à 60°C. On obtient ainsi un dérivé formylé de formule générale (V) dans laquelle R' représente un groupe formyle, que l'on réduit en présence d'hydrure double de lithium et d'aluminium, dans un solvant inerte du type éther tel que le tétrahydrofurane, à une température de 20 à 60°C.
On fait ensuite réagir ce dernier avec le 2-chloropyrimidine-4-carboxamide de formule (VI) de la manière décrite ci-dessus.

Une variante du procédé selon le schéma 1 consiste à utiliser, au lieu de l'époxyde de formule (III), l'alcool de formule (III')

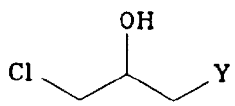

(III')

dans laquelle Y est tel que défini précédemment. La réaction avec le composé de formule générale (II) s'effectue alors dans un solvant tel que le xylène, à la température du reflux.

L'époxyde de départ de formule générale (III) est disponible dans le commerce.
Le 2-chloropyrimidine-4-carboxamide de formule (VI) peut être préparé à partir de 2-chloropyrimidine-4-carbonitrile par traitement à l'acide chlorhydrique gazeux dans l'acide formique, ledit nitrile étant lui-même préparé selon la méthode décrite dans *J. Het. Chem.,* 1964, **1**, 130-133.

Le composé de départ de formule (III') peut être obtenu à partir du composé de formule (III) par ouverture du cycle époxyde au moyen d'acide chlorhydrique.

Si on désire obtenir un composé de formule générale (I) optiquement pur, on peut, par exemple, mettre en oeuvre une méthode classique de cristallisation fractionnée de diastéréoisomères, après avoir préparé un ester ou un carbamate du composé de formule générale (I) (dans laquelle $R_1$ représente un atome d'hydrogène) au moyen d'un acide optiquement pur.

On peut aussi mettre en oeuvre le procédé du schéma 1 avec un composé de formule générale (III') optiquement pur qu'on aura isolé, par exemple par une méthode enzymatique.

Le principe de base d'une telle méthode consiste à séparer un alcool optiquement pur et l'acétate correspondant, ayant la configuration opposée, par exemple par chromatographie sur colonne de gel de silice.

Selon une première variante on soumet le composé de formule (III') racémique à une acétylation chimique, par exemple au moyen d'anhydride acétique, puis on hydrolyse seulement l'un des deux énantiomères de l'acétate racémique ainsi obtenu, en présence d'une enzyme, et on sépare l'acétate qui n'a pas été hydrolysé. On obtient un alcool optiquement pur et un acétate optiquement pur, de configuration opposée, qui peut, si on le désire, être lui même hydrolysé par voie chimique pour fournir le second énantiomère de l'alcool.

Selon une seconde variante on soumet le composé de formule (III') racémique à une acétylation stéréospécifique en présence d'une enzyme qui catalyse l'estérification d'un seul des énantiomères, par exemple au moyen d'acétate de vinyle. Comme précédemment on obtient un alcool optiquement pur et un acétate optiquement pur, de configuration opposée, qui peut, si on le désire, être lui même hydrolysé par voie chimique pour fournir le second énantiomère de l'alcool.

Dans les deux variantes on peut, selon l'enzyme utilisée, obtenir l'énantiomère lévogyre ou dextrogyre du composé de formule (III') et son acétate de configuration opposée.

Les enzymes utilisables sont par exemple les enzymes 30 AY de Amano™ ("Lipase P"), Lipolase 100 de Novo Nordisk™, poudre acétonique de pancréas de mouton de Sigma™, esterase de foie de lapin de Sigma™, Palatase A 750 de Novo Nordisk™, Lipase OF de Sepracor™, Lipase Type I de germe de blé de Sigma™, Pig Liver Esterase de Biocatalysts™ et, de préférence, Pig Liver Esterase de Sigma™, Lipoprotein Lipase de Amano™, poudre acétonique de pancréas de porc de Sigma™, poudre acétonique de foie de porc de Sigma™.

Les exemples suivants illustrent la préparation de quelques composés selon l'invention.
Les microanalyses élémentaires et les spectres IR et RMN confirment les structures des produits obtenus. Dans les titres des exemples, les numéros de composés entre parenthèses correspondent à ceux du tableau donné plus loin.

Exemple 1 (Composé N°3)

(±)-2-[[3-[4-(5-Chloro-2-méthoxyphényl)pipérazin-1-yl]-2-hydroxypropyl]amino]pyrimidine-4-carboxamide, chlorhydrate.

1.1. (±)-2-[2-[4-(5-Chloro-2-méthoxyphényl)pipérazin-1-yl]-2-hydroxypropyl]-1*H*-isoindole-1,3(2*H*)-dione.

A une solution de 9,98 g (41,7 mmoles) de 1-(5-chloro-2-méthoxyphényl)pipérazine dans 150 ml de propan-2-ol, on ajoute 8,13 g (40 mmoles) de *N*-(2,3-époxypropyl)phtalimide et on chauffe à la température de reflux du solvant pendant 3,5 heures, puis on laisse agiter une nuit à la température ambiante. On collecte le précipité par filtration, le lave au méthanol et le sèche sous pression réduite. On obtient un solide qui fond à 136-138°c que l'on utilise tel quel dans l'étape suivante.

1.2. (±)-α-(Aminométhyl)-4-(5-chloro-2-méthoxyphényl)pipérazine-1-éthanol.

A une solution de 300 ml d'éthanol contenant le composé 1.1., on ajoute 2,5 ml, soit 2,58 g (48,4 mmoles), d'hydrate d'hydrazine puis on chauffe à la température de reflux du solvant pendant 3,5 heures. On refroidit la solution, on évapore le solvant sous pression réduite, on traite le résidu avec 70 ml d'eau et 10 ml d'acide chlorhydrique à 36%, on chauffe le mélange réactionnel pendant 1 heure à 100°C, puis on le laisse à la température ambiante pendant une nuit. On élimine l'hydrazide phtalique, insoluble dans l'eau, par filtration, on refroidit le filtrat à 0°C, on y ajoute de l'acétate d'éthyle et de la soude à 30% jusqu'à pH ≥ 8. Après plusieurs extractions à l'acétate d'éthyle, on réunit les phases organiques et les sèche sur sulfate de sodium. On évapore le solvant sous pression réduite. On obtient 9,9 g d'huile que l'on utilise telle quelle dans l'étape suivante.

1.3. (±)-2-[[3-[4-(5-Chloro-2-méthoxyphényl)pipérazin-1-yl]-2-hydroxypropyl]amino]pyrimidine-4-carboxamide, chlorhydrate.

A une solution de 4,75 g (15,84 mmoles) du composé 1.2. dans 150 ml d'acétonitrile, on ajoute 1,6 g (10,15 mmoles) de 2-chloropyrimidine-4-carboxamide et 2,4 g (17,36 mmoles) de carbonate de potassium. On agite le mélange à la température ambiante pendant 24 heures puis on le chauffe au reflux pendant 3 heures. On le concentre partiellement, sous pression réduite, on y ajoute de l'eau et on extrait le produit de la réaction au dichlorométhane.

On sèche la phase organique sur sulfate de sodium, et on la concentre sous pression réduite. On obtient une huile que l'on purifie par chromatographie sur colonne de gel de silice (éluant : dichlorométhane, puis acétate d'éthyle, puis acétate d'éthyle/méthanol 98/2 à 85/15). On isole 1,45 g d'une huile qui cristallise et que l'on fait recristalliser dans de l'acétonitrile.

Point de fusion : 141-142°C.

On prépare le chlorhydrate à partir de 1,43 g (3,39 mmoles) de base dissous dans 10 ml de dichlorométhane et 34 ml de solution 0,1N d'acide chlorhydrique dans du propan-2-ol. On concentre la solution sous pression réduite et on recristallise le résidu dans de l'acétone. On obtient 1,4 g de chlorhydrate.

Point de fusion : 231-235°C (décomposition).

Exemple 2 (Composé N°5)

(±)-2-[[3-[4-(5-Chloro-2-méthoxyphényl)pipérazin-1-yl)-2-hy-droxypropyl]méthylamino]pyrimidine-4-carboxamide, chlorhydrate.

2.1. (±)-*N*-[3-[4-(5-Chloro-2-méthoxyphényl)pipérazin-1-yl]-2-hydroxypropyl]formamide.

On chauffe une solution de 5,15 g (17,01 mmoles) du composé 1.2. dans 50 ml de formiate d'éthyle, à la température du reflux, pendant 5 heures, puis on évapore le formiate d'éthyle en excès sous pression réduite.

On obtient un résidu huileux que l'on purifie par chromatographie sur colonne de gel de silice (éluant : acétate d'éthyle/méthanol 99/1 puis 90/10). On isole 5,10 g d'huile que l'on utilise telle quelle dans l'étape suivante.

2.2. (±)-α-[(Méthylamino)méthyl]-4-(5-chloro-2-méthoxyphényl)pipérazine-1-éthanol.

Dans un ballon de 0,5 l, on introduit successivement 0,9 g (23,7 mmoles) d'hydrure de lithium et d'aluminium, 20 ml de tétrahydrofurane sec et, goutte à goutte, une solution de 5,10 g (15,55 mmoles) de composé 2.1. dans 100 ml de tétrahydrofurane sec. On chauffe le mélange à la température du reflux pendant 5 heures, puis on le laisse revenir à la température ambiante et on hydrolyse par une solution aqueuse de soude. On extrait le produit au moyen d'éther diéthylique. On réunit les phases organiques, on les sèche sur sulfate de sodium et on les concentre. On obtient 4,54 g de résidu huileux, qu'on utilise tel quel dans l'étape suivante.

2.3. (±)-2-[[3-[4-(5-Chloro-2-méthoxyphényl)pipérazin-1-yl)-2-hydroxypropyl]méthylamino]pyrimidine-4-carboxamide, chlorhydrate.

Dans un ballon de 0,5 l, on introduit successivement 4,5 g (14,33 mmoles) du composé 2.2., 2 g (12,6 mmoles) de 2-chloropyrimidine-4-carboxamide, 2,4 g (17,36 mmoles) de carbonate de potassium et 200 ml d'acétonitrile, et on agite le mélange pendant 32 heures à la température ambiante. On le concentre sous pression réduite, on ajoute de l'eau au résidu et on extrait au dichlorométhane. On sèche la phase organique sur sulfate de sodium, puis on évapore le solvant sous pression réduite. On purifie le résidu par chromatographie sur colonne de gel de silice (éluant : dichlorométhane/méthanol de 99/1 à 85/15). On fait cristalliser le résidu d'évaporation dans de l'acétonitrile et on obtient 1,48 g de solide.

Point de fusion : 135-136°C.

On prépare le chlorhydrate à partir de 1,48 g (3,4 mmoles) de base dissous dans 10 ml de dichlorométhane et 1 ml de méthanol, et 34 ml d'acide chlorhydrique 0,1N dans du propan-2-ol. On concentre la solution et on fait cristalliser le résidu dans de l'acétone. On obtient 1,32 g de solide blanc Point de fusion : 231-235°C (décomposition).

Exemple 3 (Composé N°2b)

(+)-2-[[3-[4-(5-Fluoro-2-méthoxyphényl)pipérazin-1-yl]-2-hydroxypropyl]amino]pyrimidine-4-carboxamide, fumarate.

3.1 (-)-2-(3-chloro-2-hydroxypropyl)-1*H*-isoindole-1,3(2*H*)-dione.

On dissout 4 g (0,009 moles) d'acétate de 1-chloro-3-(1,3-di-oxo-1*H*(2*H*)-isoindol-2-yl)prop-2-yle dans 50 ml de tert.-butylméthyléther, on ajoute 200 ml de tampon phosphate (dihydrogénophosphate de potassium et dihydrogénophosphate de potassium, 0,01M, pH=7,2) puis 0,4 g de poudre acétonique de foie de porc, et on agite le mélange à température ambiante pendant 18 heures en maintenant le pH à 7,2 par addition de soude 1N à l'aide d'un pH-stat.

On filtre le mélange, on sépare la phase organique, on la lave avec un solution saturée de chlorure de sodium, on la sèche sur sulfate de sodium et on évapore le solvant sous pression réduite. On purifie le résidu par chromatographie sur colonne de gel de silice en éluant avec un mélange cyclohexane/acétate d'éthyle 70/30.

On obtient 1,5 g d'alcool riche en énantiomère dextrogyre, et 1,64 g d'acétate lévogyre pur.

Point de fusion de l'acétate : 88-90°C.

Pouvoir rotatoire : $[\alpha]_D^{20}$ = -17,8° (c=0,78 ; EtOH).

Excès énantiomérique : ee=90%.

On soumet l'acétate à une hydrolyse chimique au moyen de 10 équivalents d'acide chlorhydrique sec (chlorure d'acétyle + méthanol) pendant 24 heures, et on isole 1,03 g d'alcool lévogyre chimiquement pur.

Point de fusion : 76-78°C.

Pouvoir rotatoire : $[\alpha]_D^{20}$ = -29° (c=0,315 ; EtOH).

Excès énantiomérique : ee=90% (CLHP chirale).

3.2. (+)-2-[3-[4-(5-Fluoro-2-méthoxyphényl)pipérazin-1-yl]-2-hydroxypropyl]-1*H*-isoindole-1,3(2*H*)-dione.

On dissout 1,03 g (0,004 mole, ee=90%) de (-)-2-(3-chloro-2-hydroxypropyl)-1*H*-isoindole-1,3(2*H*)-dione et 0,84 g (0,004 mole) de 1-(5-fluoro-2-méthoxyphényl)pipérazine dans 10 ml de xylène et on chauffe le mélange à reflux en présence de traces d'iodure de sodium pendant 12 heures. On évapore le solvant et on purifie le résidu par chromatographie sur colonne de gel de silice (éluant : cyclohexane/acétate d'éthyle 60/40). On isole 0,62 g de solide blanc.

Point de fusion : 126-130°.

Pouvoir rotatoire : $[\alpha]_D^{20}$ = +41,5° (c=0,26 ; EtOH).

3.3. (+)-2-[[3-[4-(5-Fluoro-2-méthoxyphényl)pipérazin-1-yl]-2-hydroxypropyl]amino]pyrimidine-4-carboxamide, fumarate.

3.3a. On dissout 0,62 g (0,00145 mole) de (+)-2-[3-[4-(5-fluoro-2-méthoxyphényl)pipérazin-1-yl]-2-hydroxypropyl]-1*H*-isoindole-1,3(2*H*)-dione dans 40 ml d'éthanol contenant 0,15 ml (0,003 mole) d'hydrate d'hydrazine et on chauffe le mélange au reflux pendant 4 heures. On évapore le mélange à sec et on reprend le résidu avec 10 ml d'eau et 2,5 ml d'acide chlorhydrique concentré. On chauffe le mélange à reflux pendant 1,5 heure, on le laisse refroidir, et on élimine l'insoluble par filtration. On traite le filtrat avec une solution aqueuse de soude à 30% jusqu'à pH≥8, et on extrait à l'acétate d'éthyle ; après séchage de la phase organique et évaporation du solvant, on obtient 0,41 g d'huile jaune qu'on utilise telle quelle dans l'étape suivante.

3.3b. A une solution de 0,41 g (0,0014 mole) du composé précédent dans 10 ml de *N,N*-diméthylformamide, on ajoute 0,241 g (0,00153 mole) de 2-chloropyrimidine-4-carboxamide et 0,347 g (0,0025 mole) de carbonate de potassium et un cristal d'iodure de sodium. On chauffe le mélange à 50°C pendant 5 heures, sous argon. On évapore le solvant, on reprend le résidu avec de l'acétate d'éthyle, on lave la solution à l'eau, on sèche la phase organique sur sulfate de magnésium et on évapore le solvant. On obtient 0,76 g de produit sous forme d'huile que l'on purifie par chromatographie sur gel de silice (éluant : dichlorométhane/méthanol 97/3).

On obtient 0,5 g de base.

Pour préparer le fumarate on dissout à chaud 0,5 g de base (0,00123 mole) dans 20 ml d'éthanol et on ajoute à cette solution 0,143 g (0,00123 mole) d'acide fumarique dissous dans 8 ml d'éthanol. On concentre la solution à 90% du volume initial et, au résidu, on ajoute de l'acétate d'éthyle jusqu'à précipitation. On filtre l'insoluble et on le reprend à chaud dans du méthanol en présence de noir animal. Après filtration, le composé recristallise, et on obtient 0,325 g de fumarate neutre.

Point de fusion : 163-167°C

Pouvoir rotatoire : $[\alpha]_D^{20}$ = +10,5° (c=0,53 ; EtOH)

Exemple 4 (Composé N°2a)

(-)-2-[[3-[4-(5-Fluoro-2-méthoxyphényl)pipérazin-1-yl]-2-hydroxypropyl]amino]pyrimidine-4-carboxamide, fumarate.

4.1. (+)-2-(3-chloro-2-hydroxypropyl)-1*H*-isoindole-1,3(2*H*)-dione.

On soumet les 1,5 g (0,00626 moles) d'alcool riche en énantiomère dextrogyre, obtenu dans l'exemple 3.1, à une acétylation au moyen de 3,77 g (0,0438 moles) d'acétate de vinyle, en présence de 3,167 g (0,0313 moles) de triéthylamine et de 0,8 g de lipase P dans 25 ml de tétrahydrofurane pendant 4 jours à température ambiante.

Après purification par chromatographie sur colonne de gel de silice (éluant : hexane/acétate d'éthyle 70/30) on isole finalement 1,02 g d'alcool dextrogyre chimiquement pur.

Pouvoir rotatoire : $[\alpha]_D^{20}$ = +26,2° (c=0,31 ; EtOH).

Excès énantiomérique : ee=85% (CLHP chirale).

4.2. (-)-2-[3-[4-(5-Fluoro-2-méthoxyphényl)pipérazin-1-yl]-2-hydroxypropyl]-1*H*-isoindole-1,3(2*H*)-dione.

On dissout 1,02 g (0,004 mole, ee=85 %) de (+)-2-(3-chloro-2-hydroxypropyl)-1*H*-isoindole-1,3(2*H*)-dione et 0,84 g (0,004 mole) de 1-(5-fluoro-2-méthoxyphényl)pipérazine dans 10 ml de xylène et on chauffe le mélange à reflux en présence de traces d'iodure de sodium, pendant 12 heures.

On évapore le solvant sous pression réduite et on purifie le résidu par chromatographie sur colonne de gel de silice (éluant : dichlorométhane/méthanol 90/10). On obtient 1,3 g de solide jaune que l'on utilise tel quel dans l'étape suivante.

4.3. (-)-2-[[3-[4-(5-Fluoro-2-méthoxyphényl)pipérazin-1-yl]-2-hydroxypropyl]amino]pyrimidine-4-carboxamide, fumarate.

4.3a. On dissout 1,3 g du composé précédent dans 40 ml d'éthanol et on chauffe à reflux pendant 1,5 heure en présence de 0,157 g (0,003 mole) d'hydrate d'hydrazine. On concentre le mélange sous pression réduite et on traite le résidu par 20 ml d'eau et 5 ml d'acide chlorhydrique concentré. On chauffe le mélange au reflux pendant 1,5 heure et on le refroidit, on élimine l'insoluble par filtration et on traite le filtrat par une solution de soude à 30% jusqu'à pH≥8. On décante la phase organique, on la sèche et on évapore le solvant sous pression réduite. On obtient 0,35 g d'une huile jaune qu'on utilise telle quelle dans l'étape suivante.

4.3b. A une solution de 0,35 g (0,0012 mole) du composé précédemment obtenu dans 20 ml de *N,N*-diméthylformamide, on ajoute 0,207 g (0,00132 mole) de 2-chloropyrimidine-4-carboxamide et 0,273 g (0,0021 mole) de carbonate de potassium et un cristal d'iodure de sodium. On chauffe le mélange à 50°C pendant 5 heures sous argon. On évapore le solvant sous pression réduite, et on obtient une huile que l'on purifie par chromatographie sur colonne de gel de silice (éluant : dichlorométhane/méthanol 97/3). On isole 0,290 g de base dont on prépare le fumarate en la dissolvant dans 10 ml d'éthanol, à chaud, et en ajoutant 0,083 g (0,00072 mole) d'acide fumarique dissous dans 4 ml d'éthanol. On concentre la solution sous pression réduite à 90 % de son volume initial et on ajoute au résidu de l'acétate d'ethyle jusqu'à précipitation. On filtre le solide et on le sèche.

Point de fusion : 161-163°C.

Pouvoir rotatoire : $[\alpha]_D^{20}$ = -8,25° (c=0,57 ; EtOH) .

Le tableau suivant illustre les structures chimiques et les propriétés physiques de quelques composés de l'invention.

Tableau

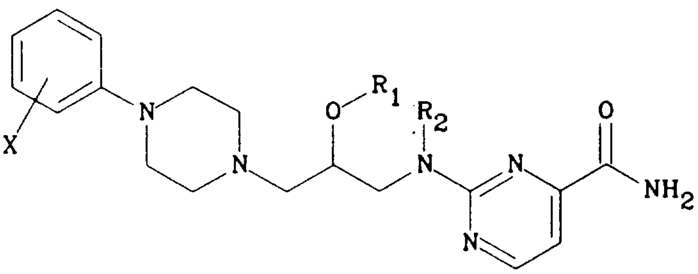

(I)

| N° | X | $R_1$ | $R_2$ | Sel | F(°C) |
|---|---|---|---|---|---|
| 1 | 3-Cl | H | H | HCl | 249-250 |
| 2 | 5-F, 2-$OCH_3$ | H | H | ½fum | 199,5-202,5 |
| 2a | $[\alpha]_D^{25}$ = -8,25° (c=0,57 ; $H_2O$) | | | fum | 161-163 |
| 2b | $[\alpha]_D^{25}$ = +10,5° (c=0,53 ; $H_2O$) | | | ½fum | 163-167 |
| 3 | 5-Cl, 2-$OCH_3$ | H | H | HCl | 231-235(dcp) |
| 4 | 2-$cC_3H_5$ | H | H | HCl | 253-254 |
| 5 | 5-Cl, 2-$OCH_3$ | H | $CH_3$ | HCl | 231-235(dcp) |
| 6 | 2-$cC_3H_5$ | H | $CH_3$ | HCl | 194-198 |

Légende

Les composés 2a et 2b sont les énantiomères respectivement lévogyre et dextrogyre du composé 2 ; dans la colonne "X", $cC_3H_5$ désigne un groupe cyclopropyle ; dans la colonne "Sel", "HCl" désigne un chlorhydrate, "fum" désigne un fumarate acide et "½fum" désigne un fumarate neutre ; dans la colonne "F(°C)", "(dcp)" désigne un point de fusion avec décomposition.

Les composés de l'invention ont fait l'objet d'études quant à leur activité antagoniste des récepteurs $\alpha_1$-adrénergiques au niveau du bas appareil urinaire.

Leur activité *in vitro* a été étudiée sur l'urètre isolé de lapin.

On prépare des anneaux d'urètre de lapin adulte selon la méthode de Ueda et al., *Eur. J. Pharmacol.,* (1984), **103**,

249-254, puis, après sensibilisation à la noradrénaline, on détermine la courbe concentration-réponse à la phényléphrine, en absence et en présence de composé à étudier.
On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.
Les $pA_2$ des composés sont de l'ordre de 5,5 à 9.

L'activité *in vivo* des composés de l'invention a été étudiée quant à leur effet sur l'hypertonie urétrale engendrée par la stimulation des fibres sympathiques du nerf hypogastrique chez le chat anesthésié.
On anesthésie des chats mâles adultes au pentobarbital sodique, et on les prépare selon la méthode de Theobald, *J. Auton. Pharmac.,* (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme $\alpha_1$-adrénergique de chaque composé par calcul de la $DI_{50}$, dose qui inhibe de 50% l'hypertonie urétrale.
Les $DI_{50}$ des composés de l'invention sont de l'ordre de 0,01 à 1 mg/kg.

Les résultats des essais montrent que les composés de l'invention montrent, *in vitro,* une activité antagoniste des récepteurs $\alpha_1$-adrénergiques des muscles lisses du bas appareil urinaire (urètre) stimulés par un agoniste $\alpha_1$-adrénergique (phényléphrine). *In vivo,* ils inhibent l'hypertonie urétrale engendrée par la stimulation nerveuse sympathique.

Les composés de l'invention peuvent donc être utilisés pour le traitement symptomatique des maladies et affections impliquant une hyperactivité du système α-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 500 mg de substance active.

**Revendications**

**Revendications pour les Etats contractants suivants : AT, BE, CH, DK, DE, FR, GB, IT, LI, LU, MC, NL, PT, SE**

1. Dérivé de pyrimidine-4-carboxamide, sous la forme d'énantiomère pur ou de mélange d'énantiomères, répondant à la formule générale (I)

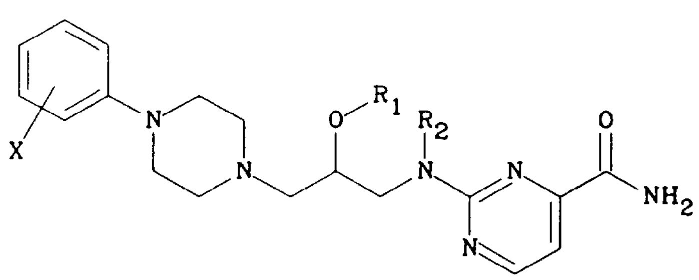

(I)

dans laquelle

X représente un ou plusieurs atomes et/ou groupes choisis parmi les suivants : hydrogène, fluor, chlore, méthoxy et cyclopropyle,
$R_1$ représente un atome d'hydrogène
et
$R_2$ représente un atome d'hydrogène ou un groupe méthyle,

ainsi que ses sels d'addition à des acides pharmaceutiquement acceptables.

2. Procédé de préparation des composés selon la revendication 1, caractérisé en ce que l'on fait réagir une pipérazine de formule générale (II)

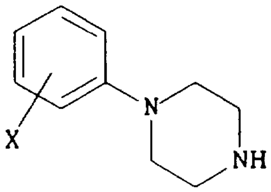

(II)

dans laquelle X est tel que défini ci-dessus, avec un réactif de formule (III) ou (III'),

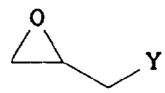

(III)

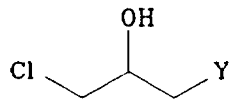

(III')

dans laquelle Y représente un groupe phtalimido, puis on traite le dérivé de formule générale (IVa) ainsi obtenu

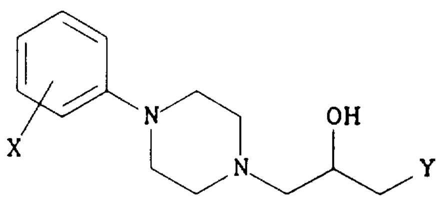

(IVa)

avec de l'hydrate d'hydrazine, puis par de l'acide chlorhydrique aqueux, pour obtenir le chlorhydrate de l'amine de formule générale (V),

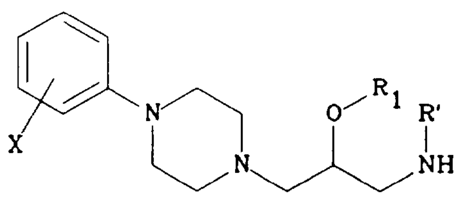

(V)

dans laquelle $R_1$ et R' représentent chacun un atome d'hydrogène,
puis, si l'on désire un composé de formule générale (I) dans laquelle $R_2$ représente un groupe méthyle, on traite le composé de formule générale (V) par un formiate d'alkyle, puis on réduit le dérivé formylé de formule générale (V) (dans laquelle R' représente un groupe formyle) en présence d'hydrure double de lithium et d'aluminium,
et enfin
on fait réagir le composé de formule générale (V) (dans laquelle R' représente un atome d'hydrogène ou un groupe méthyle) avec le 2-chloropyrimidine-4-carboxamide.

**3.** Médicament, caractérisé en ce qu'il consiste en un composé selon la revendication 1.

**4.** Composition pharmaceutique, caractérisée en ce qu'elle contient un composé selon la revendication 1 en association avec tout excipient approprié.

**Revendication pour les Etats contractants suivants : ES, GR**

**1.** Procédé de préparation d'un dérivé de pyrimidine-4-carboxamide répondant à la formule générale (I)

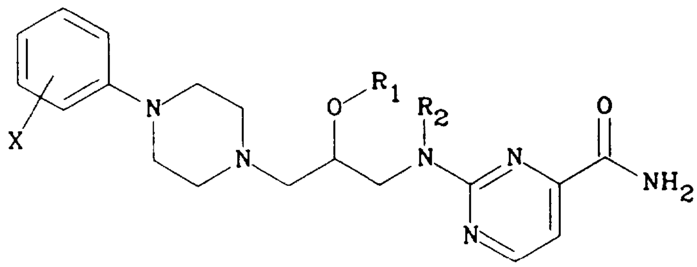

(I)

dans laquelle

X représente un ou plusieurs atomes et/ou groupes choisis parmi les suivants : hydrogène, fluor, chlore, méthoxy et cyclopropyle,
$R_1$ représente un atome d'hydrogène
et
$R_2$ représente un atome d'hydrogène ou un groupe méthyle,

procédé caractérisé en ce que l'on fait réagir une pipérazine de formule générale (II)

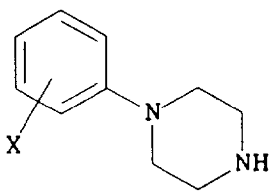

(II)

dans laquelle X est tel que défini ci-dessus, avec un réactif de formule (III) ou (III'),

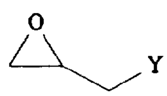

(III)

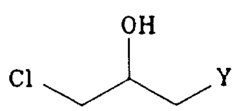

(III')

dans laquelle Y représente un groupe phtalimido, puis on traite le dérivé de formule générale (IVa) ainsi obtenu

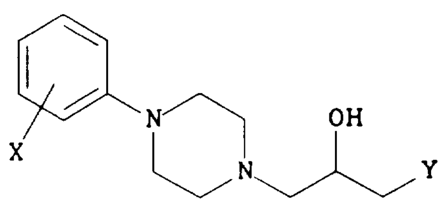

(IVa)

avec de l'hydrate d'hydrazine, puis par de l'acide chlorhydrique aqueux, pour obtenir le chlorhydrate de l'amine de formule générale (V),

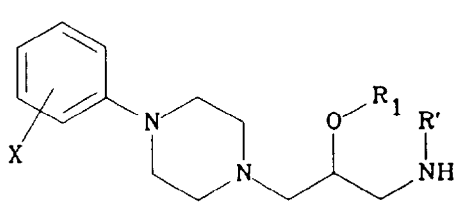

(V)

dans laquelle $R_1$ et R' représentent chacun un atome d'hydrogène,
puis, si l'on désire un composé de formule générale (I) dans laquelle $R_2$ représente un groupe méthyle, on traite le composé de formule générale (V) par un formiate d'alkyle, puis on réduit le dérivé formylé de formule générale (V) (dans laquelle R' représente un groupe formyle) en présence d'hydrure double de lithium et d'aluminium,

et enfin
on fait réagir le composé de formule générale (V) (dans laquelle R' représente un atome d'hydrogène ou un groupe méthyle) avec le 2-chloropyrimidine-4-carboxamide.

## Patentansprüche

**Patentansprüche für folgende Vertragsstaaten : AT, BE, CH, DK, DE, FR, GB, IT, LI, LU, MC, NL, PT, SE**

1. Pyrimidin-4-carboxamid-Derivat in Form des reinen Enantiomeren oder einer Mischung von Enantiomeren der allgemeinen Formel (I)

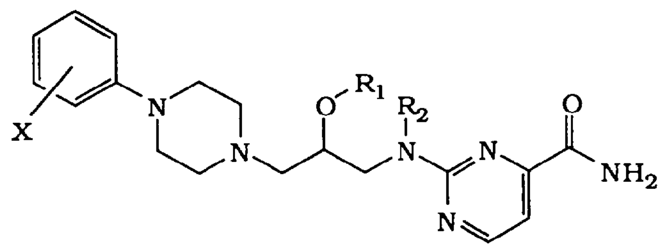

(I)

in der

X eines oder mehrere Atome und/oder Gruppen ausgewählt aus Wasserstoff, Fluor, Chlor, Methoxy und Cyclopropyl,
$R_1$ ein Wasserstoffatom und
$R_2$ ein Wasserstoffatom oder eine Methylgruppe bedeuten,

sowie deren Additionssalze mit pharmazeutisch annehmbaren Säuren.

2. Verfahren zur Herstellung der Verbindungen nach Anspruch 1, **dadurch gekennzeichnet,** daß man ein Piperazin der allgemeinen Formel (II)

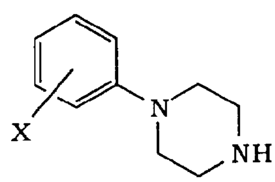

(II)

in der X die oben angegebenen Bedeutungen besitzt, mit einem Reagens der Formel (III) oder (III')

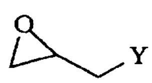

(III)

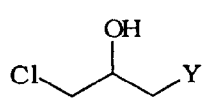

(III')

in der Y eine Phthalimidogruppe darstellt, umsetzt,
dann das in dieser Weise erhaltene Derivat der allgemeinen Formel (IVa)

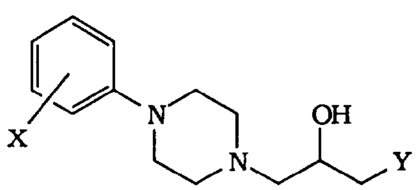

(IVa)

mit Hydrazinhydrat und dann mit wäßriger Chlorwasserstoffsäure behandelt zur Bildung des Hydrochlorids des

Amins der allgemeinen Formel (V)

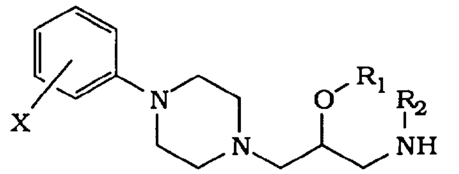

(V)

in der $R_1$ und R' jeweils ein Wasserstoffatom bedeuten,
worauf man, wenn eine Verbindung der allgemeinen Formel (I) gewünscht ist, in der $R_2$ eine Methylgruppe darstellt, man die Verbindung der allgemeinen Formel (V) mit einem Ameisensäurealkylester behandelt und dann das Formylderivat der allgemeinen Formel (V) (in der R' eine Formylgruppe darstellt) in Gegenwart von Lithiumaluminiumhydrid reduziert und schließlich
die Verbindung der allgemeinen Formel (V) (in der R' ein Wasserstoffatom oder eine Methylgruppe darstellt) mit 2-Chlorpyrimidin-4-carboxamid umsetzt.

3. Arzneimittel, **dadurch gekennzeichnet,** daß es aus einer Verbindung nach Anspruch 1 besteht.

4. Pharmazeutische Zubereitung, **dadurch gekennzeichnet,** daß sie eine Verbindung nach Anspruch 1 in Kombination mit irgendeinem geeigneten Trägermaterial enthält.

**Patentanspruch für folgende Vertragsstaaten : ES, GR**

1. Verfahren zur Herstellung eines Pyrimidin-4-carboxamid-Derivats der allgemeinen Formel (I)

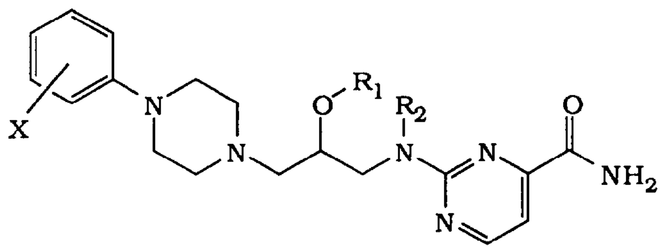

(I)

in der

X eines oder mehrere Atome und/oder Gruppen ausgewählt aus Wasserstoff, Fluor, Chlor, Methoxy und Cyclopropyl.
$R_1$ ein Wasserstoffatom und
$R_2$ ein Wasserstoffatom oder eine Methylgruppe bedeuten,

**dadurch gekennzeichnet,** daß man ein Piperazin der allgemeinen Formel (II)

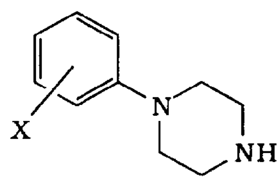

(II)

in der X die oben angegebenen Bedeutungen besitzt, mit einem Reagens der Formel (III) oder (III')

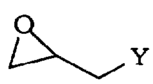

(III)

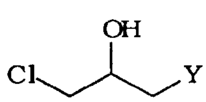

(III')

in der Y eine Phthalimidogruppe darstellt, umsetzt,
dann das in dieser Weise erhaltene Derivat der allgemeinen Formel (IVa)

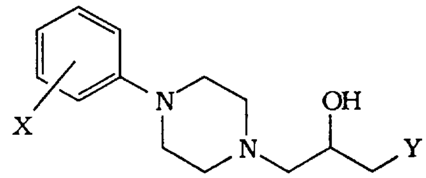

(IVa)

mit Hydrazinhydrat und dann mit wäßriger Chlorwasserstoffsäure behandelt zur Bildung des Hydrochlorids des Amins der allgemeinen Formel (V)

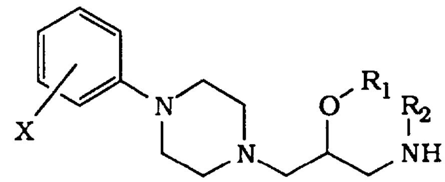

(V)

in der $R_1$ und R' jeweils ein Wasserstoffatom bedeuten,
worauf man, wenn eine Verbindung der allgemeinen Formel (I) gewünscht ist, in der $R_2$ eine Methylgruppe darstellt, man die Verbindung der allgemeinen Formel (V) mit einem Ameisensäurealkylester behandelt und dann das Formylderivat der allgemeinen Formel (V) (in der R' eine Formylgruppe darstellt) in Gegenwart von Lithiumaluminiumhydrid reduziert und schließlich
die Verbindung der allgemeinen Formel (V) (in der R' ein Wasserstoffatom oder eine Methylgruppe darstellt) mit 2-Chlorpyrimidin-4-carboxamid umsetzt.

## Claims

### Claims for the following Contracting States : AT, BE, CH, DK, DE, FR, GB, IT, LI, LU, MC, NL, PT, SE

**1.** 4-Pyrimidinecarboxamide derivative, in the form of a pure enantiomer or of a mixture of enantiomers, corresponding to the general formula (I)

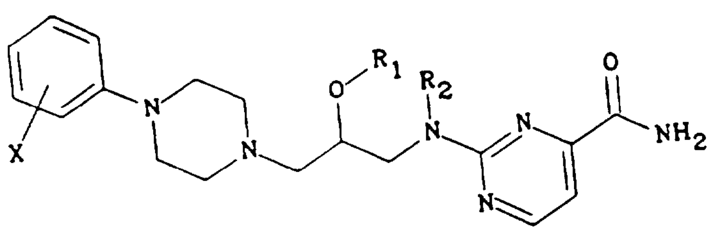

(I)

in which

X represents one or more atoms and/or groups chosen from the following: hydrogen, fluorine, chlorine, methoxy and cyclopropyl,
$R_1$ represents a hydrogen atom,
$R_2$ represents a hydrogen atom or a methyl group,

as well as its addition salts with pharmaceutically acceptable acids.

**2.** Process for preparing the compounds according to Claim 1, characterised in that a piperazine of general formula (II)

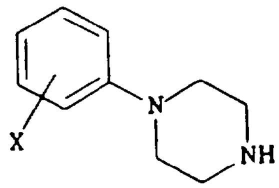

(II)

in which X is as defined above, is reacted with a reactant of formula (III) or (III'),

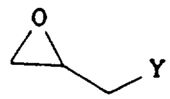

(III)

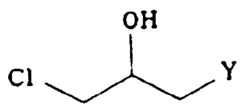

(III')

in which Y represents a phthalimido group, the derivative of general formula (IVa) thereby obtained

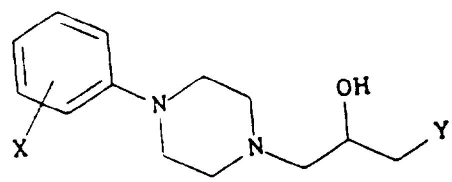

(IVa)

is then treated with hydrazine hydrate and then with aqueous hydrochloric acid to obtain the hydrochloride of the amine of general formula (V),

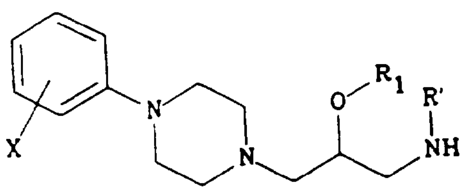

(V)

in which $R_1$ and R' each represent a hydrogen atom,
then, if a compound of general formula (I) in which $R_2$ represents a methyl group is desired, the compound of general formula (V) is treated with an alkyl formate, the formyl derivative of general formula (V) (in which R' represents a formyl group) is then reduced in the presence of lithium aluminium hydride,
and finally
the compound of general formula (V) (in which R' represents a hydrogen atom or a methyl group) is reacted with 2-chloro-4-pyrimidinecarboxamide.

**3.** Medicinal product, characterised in that it consists of a compound according to Claim 1.

**4.** Pharmaceutical composition, characterised in that it contains a compound according to Claim 1 in combination with any suitable excipient.

**Claim for the following Contracting States : ES, GR**

**1.** Process for preparing a 4-Pyrimidinecarboxamide derivative corresponding to the general formula (I)

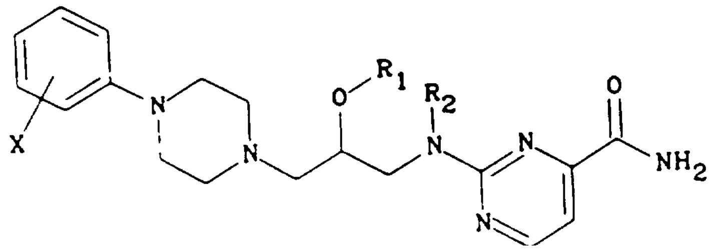

(I)

in which

X represents one or more atoms and/or groups chosen from the following: hydrogen, fluorine, chlorine, methoxy and cyclopropyl,
$R_1$ represents a hydrogen atom, and
$R_2$ represents a hydrogen atom or a methyl group,

which process is characterised in that a piperazine of general formula (II)

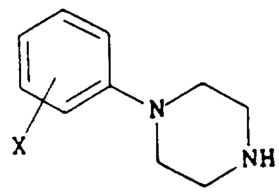

(II)

in which X is as defined above, is reacted with a reactant of formula (III) or (III'),

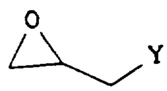

(III)

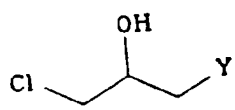

(III')

in which Y represents a phthalimido group,
the derivative of general formula (IVa) thereby obtained

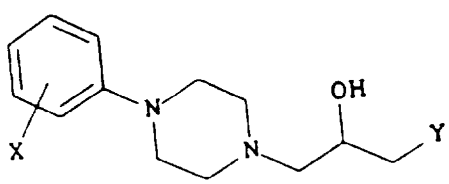

(IVa)

is then treated with hydrazine hydrate and then with aqueous hydrochloric acid to obtain the hydrochloride of the amine of general formula (V),

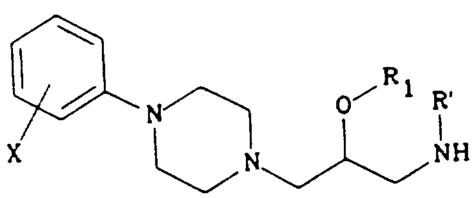

(V)

in which $R_1$ and R' each represent a hydrogen atom,
then, if a compound of general formula (I) in which $R_2$ represents a methyl group is desired, the compound of general formula (V) is treated with an alkyl formate, the formyl derivative of general formula (V) (in which R' represents a formyl group) is then reduced in the presence of lithium aluminium hydride,
and finally
the compound of general formula (V) (in which R' represents a hydrogen atom or a methyl group) is reacted with

2-chloro-4-pyrimidinecarboxamide.